# EUROPEAN PATENT SPECIFICATION

(11) **EP 4 294 336 B1**
(45) Date of publication and mention of the grant of the patent: **10.09.2025**
(21) Application number: 22710221.7
(22) Date of filing: 18.02.2022
(51) Int. Cl.: A61F 5/44, A61F 5/441, A61F 5/451, A61M 1/00

(54) **PORTABLE FLUID COLLECTION PAD FOR USE WITH FLUID COLLECTION SYSTEMS AND RELATED METHODS**
TRAGBARES FLÜSSIGKEITSSAMMELKISSEN ZUR VERWENDUNG MIT FLÜSSIGKEITSSAMMELSYSTEMEN UND ZUGEHÖRIGE VERFAHREN
TAMPON DE COLLECTE DE FLUIDE PORTABLE DESTINÉ À ÊTRE UTILISÉ AVEC DES SYSTÈMES DE COLLECTE DE FLUIDE ET MÉTHODES ASSOCIÉES

(30) Priority: 18.02.2021 US 202163150640 P
(43) Date of publication of application: 27.12.2023
(73) Proprietor: Purewick Corporation, El Cajon, California 92020 (US)
(72) Inventor: YOUNG JOYNER, Melissa, Stone Mountain, Georgia 30087 (US); ABDELAL, Dana Ahmad, Marietta, Georgia 30067 (US); KULKARNI, Vinayaka, Bangalore 56007 (IN); EARNSHAW, Audrey, Erie, Colorado 80516 (US); CHANCY, Patrick Hudson, Dunwoody, Georgia 30338 (US)
(74) Representative: Studio Torta S.p.A.
(86) International application number: PCT/US2022/016942
(87) International publication number: WO 2022/178229

(56) References cited:
- WO-A2-91/04714
- IT-A1- 201800 009 129
- US-A1- 2004 176 731
- US-A1- 2012 233 761
- US-A1- 2020 405 521

## Description

### BACKGROUND

An individual may have limited or impaired mobility such that typical urination processes are challenging or impossible. For example, the individual may have surgery or a disability that impairs mobility. In another example, the individual may have restricted travel conditions such as those experience by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections. Conventional fluid collection devices also may be limited to use when a patient is confined to a bed in a supine position. Examples of fluid collection devices are disclosed in US20200405521 A1 on which the preamble of claim 1 is based, and in US2004176731A1, IT201800009129A1, US20120233761A1.

Thus, users and manufacturers of fluid collection devices continue to seek new and improved devices, systems, and methods to collect fluid.

### SUMMARY

The invention is set out in the appended set of claims.

Embodiments disclosed herein are related to fluid collection systems. In an embodiment, a fluid collection system includes a fluid collection pad for use with a fluid collection device for collecting fluid from a subject. The fluid collection pad includes a cushion, a fluid collection container including an absorbent portion disposed therein, and a tube having a first portion configured to couple to the fluid collection device and a second portion disposed within the fluid collection container. The second portion of the tube is perforated and is disposed in a serpentine arrangement throughout the fluid collection container. The fluid collection container is configured to be disposed within the cushion.

In some embodiments, a portable fluid collection system may include a fluid collection device configured to be positioned at least proximate to a urethra of a user, the above described fluid collection pad, and a pump. The pump may be in fluid communication with the fluid collection pad and the fluid collection device. The pump may be configured to draw fluid from the fluid collection device through the tube and into the fluid collection container.

In an embodiment, a method of assembling a portable fluid collection system includes detachably securing a fluid collection pad to at least one of a chair, a wheelchair, or a bed. The method may also include positioning a fluid collection device proximate to a urethra of a user and fluidly coupling the fluid collection device to the above described fluid collection pad. The method also includes coupling a pump to the first portion of the tube, where the pump is configured to draw fluid from the fluid collection device through the tube and into the fluid collection container.

Features from any of the disclosed embodiments may be used in combination with one another as long as the resulting combination falls under the scope of the claims. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG.** 1 is a block diagram of a portable fluid collection system, according to an embodiment.
**FIG. 2A** is an isometric view of a portable fluid collection system secured to a wheelchair, according to an embodiment.
**FIG. 2B** is an isometric view of the portable fluid collection system of **FIG. 2A** without the wheelchair.
**FIG. 2C** is an isometric view of a fluid collection pad, a cushion cover, and a pump coupled to a wheelchair, according to an embodiment.
**FIG. 2D** is an isomeric view of a fluid collection pad and a cushion cover, according to an embodiment.
**FIG. 2E** is a schematic view of a pump, according to an embodiment.
**FIG. 3A** is a partial cutaway, front isometric view of a fluid collection pad, according to an embodiment.
**FIG. 3B** is a cross-section view of the fluid collection pad of **FIG. 3A****.**
**FIG. 4** is a flow diagram of a method for assembling a portable fluid collection system, according to an embodiment.

### DETAILED DESCRIPTION

Embodiments disclosed herein are related to fluid collection systems and related methods. Many users of fluid collection devices may have limited mobility, often relying on wheelchairs as a primary mode of transportation. Many users also may spend a significant amount of their day in a seated or supine position. Users and caregivers, then, are benefited from a fluid collection system that may be both discrete and mobile, allowing users to use the fluid collection system to collect fluid both at home and on the go.

In many embodiments described herein, a fluid collection system is compact and configured to be secured or mounted to a wheelchair. In some embodiments, the fluid collection system also can by positioned or placed on a surface near the user in addition to being securable or mountable to a chair, a wheelchair, or a bed. Embodiments of the fluid collection systems described herein may be mobile and discreet, allowing a user to participate in social activities without alerting others to the incontinence of the user. Embodiments of the fluid collection pad include a cushion to improve comfort in the seated position and further provide discretion. The fluid collection pad may include a fluid collection container having an absorbent portion disposed therein. The fluid collection container is configured to be disposed within the cushion. Portions of the fluid collection container, pad, and/or system may be removable and replaceable. The pad may include an odor neutralizer. Further, the fluid collection systems may include a cushion cover configured to hold at least the fluid collection pad therein to obscure the pad from view outside of a seat or bed. The fluid collection pad may be obscured from view outside the cushion and/or obscures the fluid held in the fluid collection container.

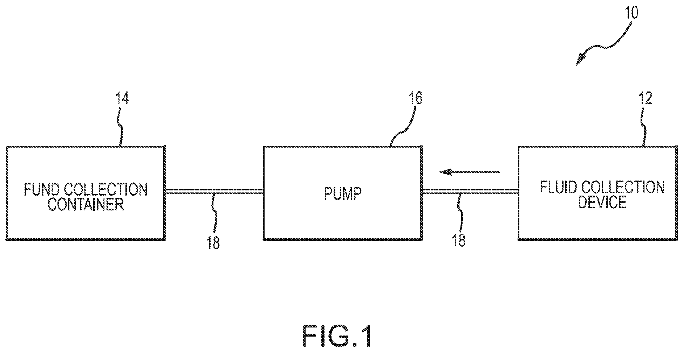

**FIG. 1** is a block diagram of a fluid collection system 10, according to an embodiment. The fluid collection system 10 may be included in any of the embodiments of fluid collection systems described herein. The system 10 includes a fluid collection device 12 (*e.g.,* any of the fluid collection assemblies disclosed herein), a fluid collection container 14, and a pump 16. The fluid collection device 10, the fluid collection container 14, and the pump 16 may be fluidly coupled to each other via one or more tubes 18 (*e.g*., medical grade tubes). For example, fluid collection device 10 may be operably coupled to one or more of the fluid collection container 14 or the pump 16 via the tube 18. In some embodiments, the pump 16 may be secured to a chair, a wheelchair, or a bed, and/or directly coupled to the tube 18 between the fluid collection device 12 and the fluid collection container 14. Fluid (*e.g.*, urine or other bodily fluids) collected in the fluid collection device 12 may be removed from the fluid collection device 12 via the tube 18 coupled to the fluid collection device 12. Suction force may be introduced into the chamber of the fluid collection device 12 via an inlet of the tube 18 responsive to suction (*e.g.,* vacuum) force applied to the tube 18.

The suction force may be applied to the tube 18 by the pump 16 either directly or indirectly. The suction force may be applied indirectly via the fluid collection container 14. For example, the outlet of the tube 18 may be disposed within or fluidly coupled to an interior region of the fluid collection container 14 and an additional tube 18 may extend from the fluid collection container 14 to the pump 16. Accordingly, the pump 16 may apply suction to the fluid collection device 12 via the fluid collection container 14. The suction force may be applied directly via the pump 16. For example, the tube 18 may be disposed within the pump 16. In some embodiments, pump may be a peristaltic pump, wherein the suction force is applied to the system 10 by compressing part of the tube 18, forcing the fluid to be pumped to move through the conduit 18. In some embodiments, the same or an additional tube 18 may extend from the pump 16 to a point outside of the fluid collection device 12, such as to the fluid collection container 14. In such examples, the pump 16 may be disposed between the fluid collection device 12 and the fluid collection container 14.

The fluid collection container 14 may be sized and shaped to retain a fluid therein. The fluid collection container 14 may include a bag (e.g., drainage bag), or any other enclosed container for storing fluid such as bodily fluid as further disclosed herein in more detail. In some examples, the tube 18 may extend from the fluid collection device 12 and attach to the fluid collection container 14 at a first point therein. An additional tube 18 may attach to the fluid collection container 14 at a second point thereon and may extend and attach to the pump 16. Accordingly, a vacuum (e.g., suction) may be drawn through fluid collection device 12 via the fluid collection container 14. Fluid, such as urine, may be drained from the fluid collection device 12 using the pump 16.

The pump 16 may include one or more of a manual vacuum pump, and electric vacuum pump, a diaphragm pump, a centrifugal pump, a displacement pump, a magnetically driven pump, a peristaltic pump, or any pump configured to produce a vacuum. The pump 16 may provide a vacuum or suction to remove fluid from the fluid collection device 12. In some examples, the pump 16 may be powered by one or more of a power cord (*e.g*., connected to a power socket), one or more batteries, or even manual power (*e.g.,* a hand operated vacuum pump). In some examples, the pump 16 may be sized and shaped to fit outside of, on, or within the fluid collection device 12. For example, the pump 16 may include one or more miniaturized pumps or one or more micro pumps. The vacuum sources disclosed herein may include one or more of a switch, a button, a plug, a remote, or any other device suitable to activate the pump 16.

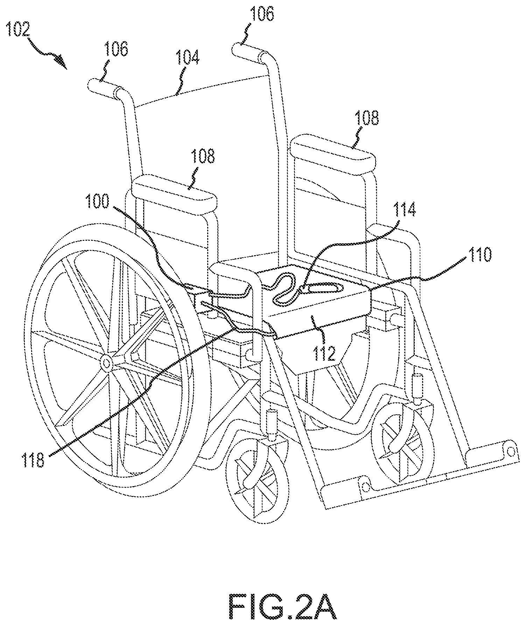

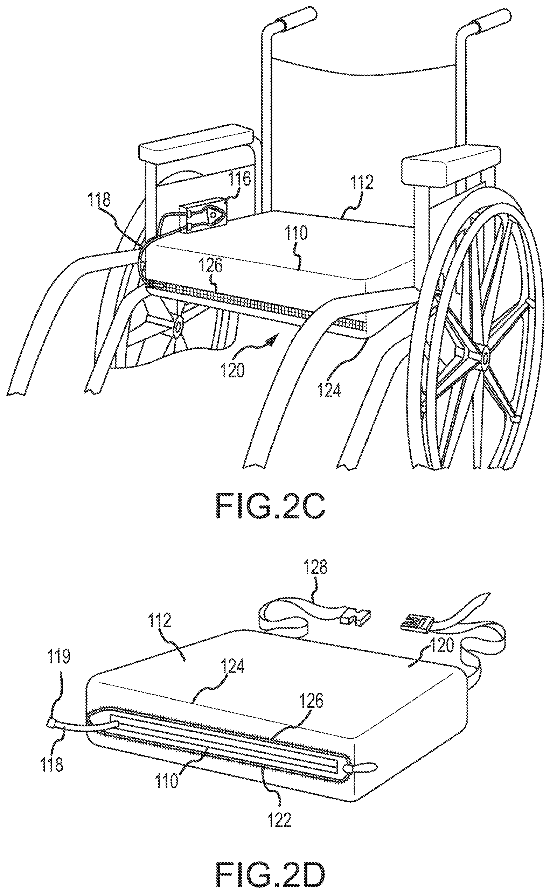

Many embodiments of fluid collection systems described herein are configured to be worn by a user, positioned on a surface such as a table, a bed, and/or securable or mountable to a bed, chair, or wheelchair. Turning to **FIG. 2A**, a fluid collection system 100 is shown secured, mounted to, or otherwise carried by a wheelchair 102. The fluid collection system 100 may be mounted to the wheelchair 102 with other supports not shown in **FIG. 2A**, such as shelves, brackets, pouches, slings, and so forth. In other embodiments, the fluid collection system 100 also may be worn by a user and/or caregiver. Whether mounted to a wheelchair 102 or worn by a user, the fluid collection system 100 may allow a user to discretely use and/or transport the fluid collection system 100.

The wheelchair 102 may include any of a number of different conventional wheelchairs, and may include, among other standard features, a back 104, two handles 106, and two arms 108. The fluid collection system 100 may include a fluid collection pad 110. In some embodiments, the fluid collection pad 110 may include a cushion 112, where the user may sit on the cushion 112, and a portion of the fluid collection system 100 is located within the cushion 112. A cushion cover or container support that is configured to detachably secure, mount, or be placed onto the wheelchair 102 and support the fluid collection system 100 may also be included.

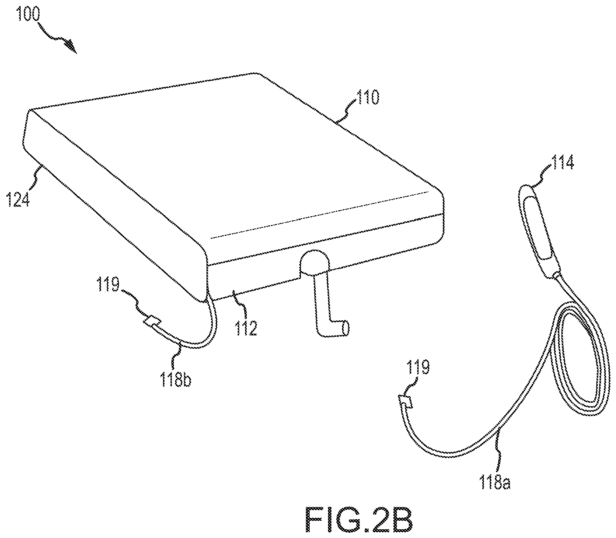

Referring now to **FIG. 2B****,** which shows components of a fluid collection system 100 removed from the wheelchair 102, according to an embodiment. The fluid collection system 100 includes a fluid collection pad 110, a cushion 112 having a fluid collection container therein (not shown), may include a cushion cover 124 or container support, a fluid collection device 114, a pump 116, and includes a tube 118. In the embodiment shown in **FIG. 2B****,** the tube 118 has a first portion 118a configured to couple to the fluid collection device 114 and a second portion 118b disposed within the fluid collection container. The fluid collection device 114 may be configured to be positioned at least proximate to a urethra of a user. While the fluid collection device 114 shown in **FIG. 2B** includes a female fluid collection device, the fluid collection device 114 may instead include a male fluid collection device. PCT Application No. PCT/US2019/029616, for example, describes various embodiments of both male and female fluid collection devices that may be used in any of the embodiments disclosed herein. Moreover, the fluid collection device 114 may be interchangeable in the fluid collection system 100 between different types, varieties, and sizes of male or female fluid collection devices. Generally, the fluid collection device 114 may include a surface sized to be positioned proximate or adjacent to the urethra of a user and configured to wick fluid or other fluids away from the user. Fluid or other fluids may be wicked from the surface to a reservoir in the fluid collection device 114.

The tube 118 includes the first portion 118a, the second portion 118b, and at least one coupling 119. The coupling 119 may be decoupled such that the tube 118 may be disconnected. The tube 118 may be disconnected to replace the fluid collection pad 110, the pump 116, the fluid collection device 114, or any other component of the fluid collection system 100. In some embodiments, the coupling 119 may be a leak-proof quick disconnect. In other embodiments, the coupling 119 may be a screw-type connector or any other suitable tubing connector.

Referring now to **FIGS. 2C-2D****,** the fluid collection pad 110 includes a cushion 112. The cushion 112 is configured to support a subject or user and provide a layer of insulation and comfort to the user. The fluid collection container 120 may be disposed within the cushion 112. The cushion 112 may be formed with foam to provide the user with a compression feel for comfort. In some embodiments, the cushion 112 may by polyurethane, layered polyurethane, reticulated foam, polyester, polyfiber, or other suitable material. In one embodiment the cushion 112 is formed from high density, viscoelastic polyurethane foam, commonly referred to as memory foam. In another embodiment, the foam may be a gel viscoelastic foam. In some embodiments, the fluid collection container 120 is disposed within the cushion 112. The cushion 112 may include a pocket 122. The pocket 122 may be sized and dimensioned to include at least a portion of the fluid collection container 120 therein. In other embodiments, the fluid collection container 120 may be configured adjacent to the cushion 112.

The fluid collection pad 110 may be disposed in a cushion cover 124. The cushion cover 124 may include an opening 126 to access the fluid collection pad 110, where the fluid collection pad 110 is disposed within the cushion cover 124. The opening 126 may be a sleeve, zipper, hook and loop fastener materials, or any other suitable opening style or method that may allow access to and/or replacement of the fluid collection pad 110. In some embodiments, the cushion cover 124 may include other openings or apertures for tubing to access and/or couple with the fluid collection pad 110. The cushion cover 124 may be constructed of any suitable material, and may include cotton, nylon, vinyl, leather, cloth, or any other upholstery material. The cushion cover may be removable and washable. In some embodiments, the cushion cover 124 may be sized and dimensioned to include at least a portion of the fluid collection pad 110 therein. In some embodiments, the entire fluid collection pad 110 may be included within the cushion cover 124.

The cushion cover 124 may include one or more straps 128 configured to secure to the wheelchair 102, a chair, or a bed. The straps 128 may include one or more fasteners configured to adjustably secure the straps 128 to the wheelchair 102, such as at least one of buckles, clips, and/or hook and loop fastener materials. The straps 128 may be sized and dimensioned to insert a structural member of a chair, a bed, a wheelchair, or other suitable structure there through. The straps 128 may be dimensioned to allow a user or caregiver to adjust the straps 128 to move the cushion cover 124 as required. As an alternative or in addition to the straps 128, in some embodiments, other securement devices may be employed to secure the fluid collection pad 110 to the wheelchair 102, such as button, Velcro, snap locks, or other suitable device. In some embodiments, the cushion cover 128 may be configured to be worn as a pack using the one or more straps 128 to make exchanging or replacing the fluid collection pad 110 easier.

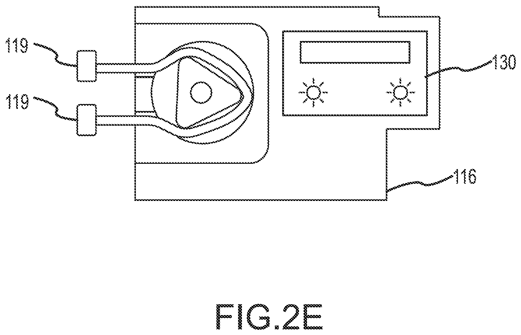

Turning now to **FIG. 2E**, in some embodiments, the fluid collection system 100 may include the pump 116. The pump 116 may be in fluid communication with the fluid collection pad 110 and fluid collection device 114. As described above generally, the pump 116 may be configured to draw fluid from the fluid collection device 114 through the tube 118 and into the fluid collection container 120. The pump 116 may be in fluid communication with the fluid collection device 114 and is configured to remove fluid from an interior region of the fluid collection device 114 and produce a suction effective to draw the fluid from the fluid collection device 114 through the tube 118 and into the fluid collection container 120.

In operation, activation of the pump 116 may pull at least a partial vacuum on the fluid collection device 114 via the tube 118 and then push the fluid to the fluid collection pad 110. In other embodiments, the pump 116 may pull an at least partial vacuum in the fluid collection container 120, which in turn pulls an at least partial vacuum on the fluid collection device 114 via the tube 118, depending on the arrangement of the pump 116 within the fluid collection system 110. In some embodiments, the vacuum created by the pump 116 pulls fluid in the fluid collection device 114 through the first portion of the tube 118a and ultimately into the fluid collection container 120 for storage and disposal. The pump 116 may be secured directly to the fluid collection container 120 or secured to a structural component of at least one of the chair, the bed, or the wheelchair. In some embodiments, the tube 118 may fluidly couple the pump 116 with the interior region of the fluid collection container 120.

The pump 116 may include one or more of a manual vacuum pump, and electric vacuum pump, a diaphragm pump, a centrifugal pump, a displacement pump, a magnetically driven pump, a peristaltic pump, or any pump configured to produce a vacuum. For example, the pump may include a peristaltic pump having a flowrate of about 25 ml/minute. The pump flowrate may include any suitable range. In some embodiments, the pump 116 may include a variable speed pump and/or a continuous pump. For example, the pump 116 may include a variable speed pump that operates at a low speed until a sensor coupled to the pump detects fluid passing through the tube 118 and into the fluid collection container 120, when the pump 116 then adjusts to a higher speed to prevent wetting or pooling of fluid at the fluid collection device 114. The pump 116 may provide a vacuum or suction to remove fluid from the fluid collection device 114. In some embodiments, the pump 116 may be powered by one or more batteries. In some examples, the pump 116 may be sized and shaped to be detachably mounted to one or more structural components of at least one of a chair, the wheelchair 102, or a bed. In some embodiments, the pump may be sized or shaped to be located within the fluid collection pad 110, such as within the pocket 122 or within the cushion cover 124. For example, the vacuum source may include one or more miniaturized pumps 116 or one or more micro pumps 116. The pump 116 may include one or more of a switch, a button, a plug, a remote, or any other device suitable to activate the pump 116.

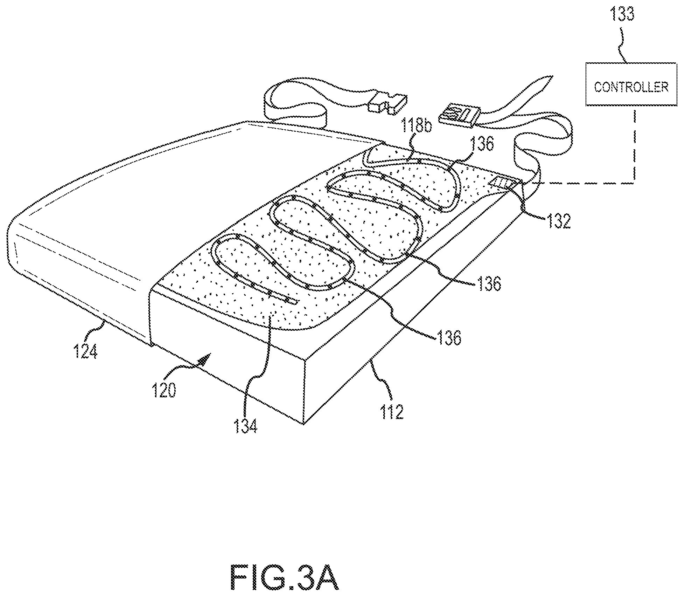

The fluid collection system 100 may also include an indicator panel 130. The indicator panel 130 may be configured to indicate at least one property related to the fluid collection system 100. In some embodiments, the indicator panel 130 may be coupled to a sensor 132 and/or a controller 133 (see FIG. 3A). The sensor 132 may take periodic or continuous readings of the property relating to the volume of the fluid in the fluid collection container 120. When the sensor 132 indicates the volume of the fluid in the fluid collection container 120 has met or exceeded a predetermined threshold (such as 75% or 90% of the total volume of the fluid collection container 120) base on the reading from the sensor 132, an electronic alert or other indication may be provided from the controller 133 to the indicator panel 130 and/or an electronic device (not shown). Upon receipt of the electronic alert, the electronic device may vibrate or beep and/or the indicator panel 130 may alarm, suggesting to the user or caregiver that the fluid collection container 120 be replaced. The user or caregiver may then disconnect the second portion of the tube 118a from the fluid collection system 100, and remove the fluid collection container 120 from the fluid collection pad 110 via the opening 126 of the cushion cover 124. The fluid collection container 120 may be removed from the cushion 112, and the user or caregiver may replace the fluid collection container 120 with a new fluid collection container and insert the new fluid collection container 120 into the pocket 122, close the opening 126 in the cushion cover 124 and reconnect the second portion of the tube 118b.

In some embodiments, based on data from the sensor 132 and the controller 133, the indicator panel 130 may provide at least one of an alarm, a display, a fluid collection container fluid saturation or fluid volume status, or may also provide a pump operating status. In some embodiments, the indicator panel 130 may be configured to communicate with the user or caregiver to empty the fluid collection container 120 when the fluid level approaches a predetermined level, to change or recharge a battery, and/or adjust a vacuum or suction level of the pump 116.

The controller 133 may transmit an alert to an electronic device that the fluid collection container 120 is at a predetermined saturation or fluid volume level, and replacing of the fluid collection container 120 is recommended. In some embodiments, the controller 133 may transmit alarms and indications, such as selected time and/or volume intervals or pump failure, a leak in the fluid collection container 120, and/or low battery to the indicator panel 130. The indicator panel 130 may include a display panel provide indications. The controller 133 may include a communication interface configured to send notifications or alerts to other electronic devices. For example, the controller 133 may be configured to send notifications or alerts at a selected radio frequency, via BLUETOOH, or via WI-FI to another electronic device and/or a mobile phone of the user or caregiver. In some embodiments, the indicator panel 130 may be included on the pump 116. In some embodiments, the controller 133 may transmit a signal wirelessly to the electronic device of the user or the caregiver and provide an indication directly to the electronic device or a smartphone. Other indications may include an alert when a battery powering at least one of the indicator panel 130 and/or the pump 116 is low. The controller 133 may wirelessly transmit an alert to the electronic device of the user or the caregiver when replacement of the fluid collection container 120 is recommended.

In some embodiments, the fluid collection system 100 may include a battery (not shown) or some other power source configured to provide power to at least one or all of the pump 116, the indicator panel 130, the sensor 132, and the controller 133. In some embodiments, the battery may include a lithium ion battery. In some embodiments, the battery may be alkaline or rechargeable. In other embodiments, the power source may be a standard electrical outlet.

Referring now to **FIG. 3A**, the fluid collection system 100 also includes tube 118 to couple the fluid collection device 114 to the pump 116 and the fluid collection container 120. The tube 118 includes the first portion 118a configured to couple to the fluid collection device 114 and the second portion 118b disposed within the fluid collection container 120. The first portion 118a and the second portion 118b may include a flexible plastic tubing. In some embodiments, at least a portion of the first portion 118a may be substantially opaque, thereby inhibiting viewing of the fluid within the first portion 118a. The second portion 118b is disposed in a serpentine arrangement throughout the fluid collection container 120. Other configurations (*e.g*. various loops) of the second portion 118b may also be implemented to disperse the fluid uniformly throughout the fluid collection container 120, such configurations not falling under the scope of the invention as claimed.

In some embodiments, the fluid collection container 120 may hold about 1 liter to about 3 liters, about 1 liter, about 2 liters, or about 3 liters of the fluid therein. The fluid collection container 120 may be generally enclosed and considered leak-proof. For example, the fluid collection container 120 may include a sealed plastic bag or a rigid plastic container. The fluid collection container 120 includes an absorbent portion 134 disposed therein. In some embodiments, the absorbent portion 134 may be sized to receive and hold therein the fluid received from the fluid collection device 114. The absorbent portion 134 may be integrated into the fluid collection container 120 and, thus, may be removably positioned within the interior region of the fluid collection pad 110 such that a user or caregiver may insert and/or remove the fluid collection container 120 that includes the absorbent portion 134. As such, a soiled absorbent portion 134 may indicate when the fluid collection container 120 should be removed from the fluid collection pad 110 and replaced with a fresh fluid collection container 120. In some embodiments, the absorbent portion 134 may be formed into a shape having two opposing generally flat or planar surfaces and generally the shape of the cushion 112. In some embodiments, the absorbent portion 134 may be formed into other shapes, sizes, and configurations. In some embodiments, the absorbent portion 134 may line and/or be secured to one or more inner surfaces of the fluid collection container 120.

The sensor 132 may be operably associated with the fluid collection container 120 and configured to detect a property relating at least to a volume of the fluid and/or absorbent portion 134 within the fluid collection container 120. In some embodiments, the sensor 132 may be located or positioned at an inlet (either interior or exterior) of the fluid collection container 120 or at a terminal end of the tube 118. The sensor 132 may be positioned along and/or within the tube 118 or placed within the absorbent portion 134, according to an embodiment. In some embodiments, the sensor 132 may include an ultrasonic sensor, a laser sensor, or an ultraviolet (UV) sensor configured to provide a continuous or periodical feedback of the property relating at least to a volume of the fluid without the sensor being in direct contact with the fluid. The sensor 132 may include a non-contact fluid sensor such as a capacitive sensor, an inductive sensor, a gravimetric sensor, or a mechanical float. The sensor 132 may be secured to the fluid collection container with at least a portion of the sensor 132 inside the interior region of the fluid collection container 120. In some embodiments, the sensor 132 may be positioned at a top of the fluid collection container 120 and directed to where the fluid collects in the interior region of the fluid collection container 120.

In some embodiments, the fluid collection system 100 may also include the controller 133. The controller 133 may be configured to communicate with the sensor 132, such as a wired or wireless connection. In some embodiments, the sensor 132 may include the controller 133. The controller 133 may include a printed circuit board (PCB) equipped with erasable programmable read-only memory (EPROM) for memory of at least data collected by the sensor 132. The controller 133 may include a processor configured to calculate a fluid level, volume, or saturation of fluid in the fluid collection container 120. The controller 133 may include a communication interface configured to send notifications or alerts to other electronic devices, such as a smartphone of the user or caregiver, an electronic device secured to the wheelchair 102 having a module and/or a display, and/or an electronic device of a healthcare system. For example, the communication interface may be configured to send notifications or alerts at a selected radio frequency, via BLUETOOH, or via WI-FI to another electronic device, such as a smartphone of the user or caregiver or an electronic device secured to the wheelchair 102. As discussed above, the controller 133 may be powered by an external or internal battery, such as a rechargeable battery.

In some embodiments, the controller 133 may be configured to wirelessly transmit an alert to an electronic device of the user or a caregiver a property relating at least to the status of the pump 116. In some embodiments, the controller 133 may be configured to operate and/or control the pump 116. In some embodiments, the controller 133 may control the pump 116 based off the status of the volume of fluid detected by the sensor 132 in the fluid collection device 114 and/or the fluid collection container 120. For example, based on data from the sensor 132, the controller 133 may start the pump 116 to cause fluid to flow from the fluid collection device 114 to the fluid collection container 120. The controller 133 may cause the pump 116 to secure when the volume of fluid in the fluid collection container 120 reaches a predetermined threshold. In some embodiments, the controller 133 may wirelessly transmit alerts and selected frequencies, such as selected time and/or volume intervals. The controller 133 may wirelessly communicate to the indicator panel 130 or the electronic device of the user or the caregiver when a battery powering at least one of the controller 133 or the pump 116 is low. The controller 133 may transmit an alert to the indicator panel 130 and/or the electronic device of the user or the caregiver when the pump 116 or fluid collection system 100 has a malfunction. In some embodiments, the controller 133 may be configured to control all operations of the pump 116.

In some embodiments, the sensor 132 may include a level transmitter configured to detect a level of the fluid in the fluid collection container 120. In some embodiments, the sensor 132 may include an ultrasonic level sensor. In an example, the sensor 132 may determine a volume of the fluid in the fluid collection container 214 using the distance between the sensor 132 and the surface of the fluid in the fluid collection container 120.

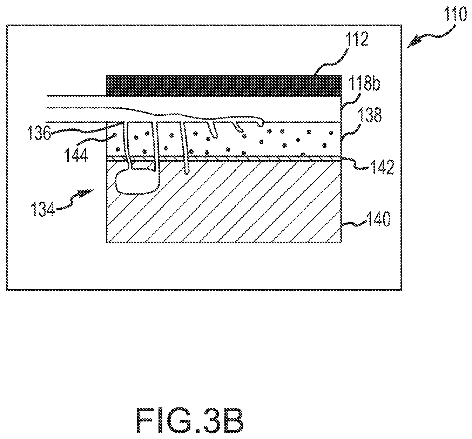

Referring now to **FIG. 3B****,** the fluid collection container 120 may be disposed underneath the cushion 112 to improve comfort for the user as they sit or lay on the fluid collection pad 110. The fluid collection container 120 may include the second portion 118b of the tube throughout the fluid collection container 120 at least in a serpentine arrangement as described above. The second portion 118b of the tube 118 is perforated. The perforations 136 of the tube 118 may be distributed to disperse the fluid uniformly throughout the fluid collection container 120 when the fluid flows through the second portion 118b. In some embodiments, the perforations 136 in the tube 118 may include holes of varying shapes, sizes, and locations to improve fluid distribution. The perforations 136 in the tube 118 may include round holes, slits, or other apertures in the tube 118 to achieve suitable fluid distribution. As fluid flows through the tube 118, the fluid may flow out of the tube 118 and into the absorbent portion 134 of the fluid collection container 120. In some embodiments, the perforations 136 may be included in any spatial arrangement to improve absorption of fluid into the fluid collection container 120. In some embodiments, the perforations 136 may be located at the top and/or bottom of the tube 118. In other embodiments, the perforations 136 may be located at the side of the tube 118 or around a circumference of the tube 118. The perforations 136 may be distributed such that the fluid is dispersed uniformly throughout the fluid collection container 120 when the fluid flows through the second portion 118b. In some embodiments, the perforations 136 may be 1/16 the diameter of the tube 118. In other embodiments, the perforations may be greater than 1/16 or less than 1/16 the diameter of the tube 118, such that flow and/or fluid distribution is improved.

The absorbent portion 134 of the fluid collection container 120 may include at least one layer including hydrophobic beads 138, at least one layer including an absorbent material 140, and a mesh layer 142 disposed between the at least one layer including hydrophobic beads and the at least one layer including an absorbent material 140. The absorbent portion 134 may be configured to disperse the fluid similar to a "french drain" or a "drainage field." The tube 118 may be disposed within the at least one layer including hydrophobic beads 138 to allow fluid to flow out of the perforations 136 without clogging the perforations 136 or the tube 118. The fluid may then seep through the hydrophobic beads 138 and into the absorbent material 140, where the fluid is absorbed. The tube 118 is disposed in a serpentine arrangement and perforations 136 distributed to disperse the fluid uniformly throughout the absorbent portion 134 to maximize the useable lifetime or capacity of the fluid collection pad 110 before it requires a replacement.

In an embodiment, the at least one layer including hydrophobic beads 138 may be located above the at least one layer including an absorbent material 140 such that gravity helps the fluid flow from the tube 118 into the absorbent material 140. In some embodiments, the second portion of the tube 118b may be disposed and/or integrated within the at least one layer of hydrophobic beads 138. The hydrophobic beads 138 may be constructed of plastic or any suitable non-absorbent material. The hydrophobic beads 138 may be at least one of latex, silica, ceramic, glass, metal, or other suitable hydrophobic material. The hydrophobic beads 138 may be disposable and/or recyclable. In some embodiments, the hydrophobic beads 138 may be configured to surround the tube 118b and aid in fluid travel throughout the absorbent portion 134 such that any absorbent material 140 does not clog the perforations 136 or otherwise impede fluid flow from the tube 118 into the absorbent material 140. In some embodiments, the bead diameter may be at least larger than the diameter of the perforations 136 and larger than any holes and/or perforations in the mesh layer 142.

In some embodiments, the absorbent material 140 may include a gelatinous starch. The absorbent material 140 may include a potato or corn starch. In some embodiments, the absorbent material 140 may include a synthetic absorbent, such as polyester or any other suitable hydrophilic absorbent material. In some embodiments, the absorbent material may include woven or nonwoven sheet material, cellulosic pulp, synthetic pulp, pulp fibers, sponge material, or other suitable synthetic or natural materials. In other embodiments, the absorbent material 140 may include a nanomaterial or nanofiber membrane configured to expand and/or form a hydrogel. In some embodiments, the absorbent material 140 may be woven into an absorbent fabric. In some embodiments, the absorbent material 140 may include a biodegradable, a non-biodegradable material, or a combination of a biodegradable and a non-biodegradable material. In some embodiments, the absorbent material 140 may be a powder-like consistency to absorb a fluid. The powder-like absorbent may be converted into a gel-like state as it absorbs fluid. In other embodiments, the absorbent material 140 may include a diaper-like absorbent material that converts the material and fluid into a gel-like state as it is collected and absorbed. The absorbent material 140 may be configured and/or constructed of a material to disperse the fluid uniformly throughout the fluid collection container 120 when the fluid flows through the second portion of the tubing 118b. The absorbent material 140 may be constructed of discrete portions of materials having more or less absorbent qualities as the tubing 118, disposed in a serpentine configuration, disperses the fluid through the perforations 136. In other words, the absorbent material 140 may include layers having differing absorbing properties to improve the capacity of the fluid collection pad 110. In some embodiments, layers of absorbent material 140 may be incorporated to improve fluid absorption or uniform distribution within the absorbent portion 134.

In some embodiments, the mesh layer 142 may be disposed between the at least one layer including the hydrophobic beads 138 and the at least one layer including the absorbent material 140. The second portion of the tube 118b may be disposed within the at least one layer of hydrophobic beads 138 and the mesh layer 142 may separate the absorbent material 140 from the tube 118 and prevent clogging the perforations 136. The mesh 142 material may act as a barrier for the absorbent material 140. The mesh layer 142 may be knitted structure of fibers woven together to create a permeable layer. In some embodiments, the mesh layer 142 may be constructed of polyester, nylon, or any other suitable material. The mesh layer 142 also may provide structure to the absorbent portion 134 and thus may be constructed of a sturdier material such as a rigid or semi-rigid plastic or metal.

The fluid collection pad 110 may include an odor neutralizer 144 configured to at least partially neutralize the odor of the fluid. In some embodiments, baking soda and/or lavender may be included to produce a more pleasant smell and neutralize odor. In some embodiments, the odor neutralizer 144 may be included in the absorbent portion 134. The odor neutralizer 144 may be integrated into the absorbent material 140 and/or the mesh layer 142.

In some embodiments, the second portion of the tube 118b may be disposed within and/or on the absorbent portion 134. In these embodiments, the layer including the hydrophobic beads 138 and/or the mesh layer may be omitted from the absorbent portion 134. The absorbent material 140 may include a material that does not gel and/or clog the perforations 136 of the second portion of the tube 118b. In some embodiments, the absorbent material 140 may wick fluid away from the second portion of the tube 118b.

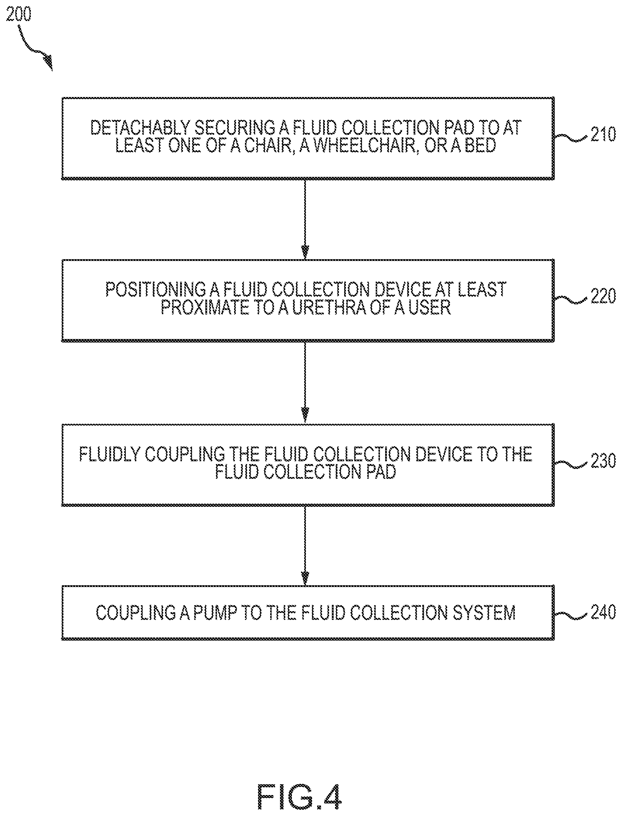

**FIG. 4** is a flow diagram of a method 200 for assembling a portable fluid collection system, according to an embodiment. The method 200 includes an act 210 of detachably securing a fluid collection pad to at least one of a chair, a wheelchair, or a bed. The method 200 also includes an act 220 of positioning a fluid collection device at least proximate to a urethra of a user. The method 200 also includes an act 230 of fluidly coupling the fluid collection device to the fluid collection pad. The fluid collection pad includes a cushion, a fluid collection container including an absorbent portion disposed therein, where the fluid collection container is configured to be disposed within the cushion, and a tube having a first portion configured to couple to a fluid collection device and a second portion disposed within the fluid collection container. The second portion of the tube is perforated.

The method also includes an act 240 of coupling a pump to the fluid collection system. In some embodiments, the pump may be coupled to the first portion of the tube. In some embodiments, the pump may be configured to draw fluid from the fluid collection device through the tube and into the fluid collection container. In some embodiments, the pump may be secured to one or more structural components of the chair, the wheelchair, or the bed. The act 240 may include mounting a pump to the chair, bed or wheelchair with the pump in fluid communication with the fluid collection container and configured to draw fluid from the fluid collection device through the first portion of the tube into the fluid collection pad.

The method 200 may include assembling any of the fluid collection systems described herein. For example, the act 210 of detachably securing a container support to the wheelchair may include securing one or more straps coupled to a cushion cover to one or more structural components of the chair, the wheelchair, or the bed. In some embodiments, the act 210 of detachably securing a fluid collection system to at least one of a chair, a wheelchair, or a bed may include positioning the fluid collection container within the cushion and placing the cushion within a cushion cover. In some embodiments, the cushion cover encloses the fluid collection pad inside a pocket. The act 210 of removing and replacing the fluid collection pad when a volume of the fluid in the fluid collection container has reached or exceeded a predetermined volume. In some embodiments, the volume of the fluid in the fluid collection container may be monitored by a sensor.

In some embodiments, the fluid collection pad may be removed from a pocket in the cushion, disconnected from the fluid collection system via a coupling on the tube, and disposed. A new fluid collection bad may then be coupled to the fluid collection system and restored to the pocket within the cushion. In some embodiments, a cushion cover having an opening may include the fluid collection pad within the cushion cover for discretion.

The acts of the method 200 described above are for illustrative purposes. For example, the acts of the method 200 can be performed in different orders, split into multiple acts, modified, supplemented, or combined, as long as the resulting method falls within the scope of claim 14. In an illustrative method, not falling under the scope of the invention as claimed, one or more of the acts of the method 200 can be omitted from the method 200. Any of the acts of the method 200 can include using any of the portable fluid collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting.

## Claims

1. A fluid collection pad for use with a fluid collection device for collecting fluid from a subject, the fluid collection pad (110) comprising:
a cushion (112);
a fluid collection container (120) including an absorbent portion (134) disposed therein, wherein the fluid collection container (120) is configured to be disposed within the cushion (112); and
a tube (118) having a first portion (118a) configured to couple to the fluid collection device (114) and a second portion (118b) disposed within the fluid collection container (120), wherein the second portion (118b) of the tube (118) is perforated; **characterized in that** the second portion (118b) of the tube (118) is disposed in a serpentine arrangement throughout the fluid collection container (120).

2. The fluid collection pad of claim 1, wherein the second portion (118b) of the tube (118) is integrated into the absorbent portion (134).

3. The fluid collection pad of any of claims 1-2, wherein the absorbent portion (134) includes:
at least one layer including hydrophobic beads (138);
at least one layer including an absorbent material (140); and
a mesh layer (142) disposed between the at least one layer including the hydrophobic beads (138) and the at least one layer including an absorbent material (140), wherein the second portion (118b) of the tube (118) is disposed within the at least one layer of hydrophobic beads (138).

4. The fluid collection pad of any of claims 1-3, wherein the fluid collection container (120) is constructed of a leak-proof material and is removable from the cushion (112) and replaceable.

5. The fluid collection pad of claim 1, further comprising an odor neutralizer (144) configured to at least partially neutralize an odor of the fluid.

6. A portable fluid collection system, comprising:
a fluid collection device (114) configured to be positioned at least proximate to a urethra of a user;
the fluid collection pad (110) according to any one of claims 1 - 5, wherein the container is disposed within the cushion (112): and
a pump (116) in fluid communication with the fluid collection pad (110) and the fluid collection device (114), wherein the pump (116) is configured to draw fluid from the fluid collection device (114) through the tube (118) and into the fluid collection container (120).

7. The fluid collection system of claim 6, wherein the second portion (118b) of the tube (118) is integrated into the absorbent portion (134).

8. The fluid collection system of claims 6 or 7, wherein the second portion (118b) of the tube (118) includes perforations (136) that are distributed to disperse the fluid uniformly throughout the fluid collection container (120) when the fluid flows through the second portion (118b).

9. The portable fluid collection system of any of claims 6-8, further comprising a cushion cover (124), wherein the fluid collection pad (110) is disposed within the cushion cover (124).

10. The portable fluid collection system of any of claims 6-9, wherein the pump (116) is coupled to the tube (118) between the fluid collection device (114) and the fluid collection container (120).

11. The portable fluid collection system of claim 6, further comprising a sensor (132) coupled to the fluid collection container (120) configured to indicate at least one property relating to the fluid collection system (100).

12. The portable fluid collection system of claim 11, wherein the sensor (132) is configured to detect a characteristic indicative of a volume of the fluid in the fluid collection container (120).

13. The portable fluid collection system of claim 6, further comprising an indicator panel, wherein the indicator panel includes at least one of an alarm, a display, a fluid collection container saturation or volume status, or a pump operating status.

14. A method of assembling a portable fluid collection system, the method comprising:
detachably securing a fluid collection pad (110) to at least one of a chair, a wheelchair (102), or a bed;
positioning a fluid collection device (114) at least proximate to a urethra of a user;
fluidly coupling the fluid collection device (114) to the fluid collection pad (110), wherein the fluid collection pad (110) includes:
a cushion (112);
a fluid collection container (120) including an absorbent portion (134) disposed therein, wherein the fluid collection container (120) is configured to be disposed within the cushion (112); and
a tube (118) having a first portion (118a) configured to couple to a fluid collection device (114) and a second portion (118b) disposed within the fluid collection container (120), wherein the second portion (118b) of the tube (118) is perforated and is disposed in a serpentine arrangement throughout the fluid collection container (120); and
coupling a pump (116) to the first portion (118a) of the tube (118), wherein the pump (116) is configured to draw fluid from the fluid collection device (114) through the tube (118) and into the fluid collection container (120).

15. The method of claim 14, wherein detachably securing a fluid collection system to at least one of a chair, a wheelchair (102), or a bed includes positioning the fluid collection container (120) within the cushion (112) and placing the cushion (112) within a cushion cover (124), wherein the cushion cover (124) encloses the fluid collection pad (110) inside a pocket; and
removing and replacing the fluid collection pad (110) when a volume of the fluid in the fluid collection container (120) has reached or exceeded a predetermined volume.

## Patentansprüche

1. Flüssigkeitssammelpad zur Verwendung mit einer Flüssigkeitssammelvorrichtung zum Sammeln von Flüssigkeit von einem Subjekt, wobei das Flüssigkeitssammelpad (110) umfasst:
ein Kissen (112);
einen Flüssigkeitssammelbehälter (120) mit einem darin angeordneten absorbierenden Abschnitt (134), wobei der Flüssigkeitssammelbehälter (120) so konfiguriert ist, dass er innerhalb des Kissens (112) angeordnet werden kann; und
einen Schlauch (118) mit einem ersten Abschnitt (118a), der so konfiguriert ist, dass er mit der Flüssigkeitssammelvorrichtung (114) gekoppelt werden kann, und einem zweiten Abschnitt (118b), der innerhalb des Flüssigkeitssammelbehälters (120) angeordnet ist, wobei der zweite Abschnitt (118b) des Schlauchs (118) perforiert ist; **dadurch gekennzeichnet, dass** der zweite Abschnitt (118b) des Schlauchs (118) in einer serpentinenförmigen Anordnung durch den gesamten Flüssigkeitssammelbehälter (120) angeordnet ist.

2. Flüssigkeitssammelpad nach Anspruch 1, wobei der zweite Abschnitt (118b) des Schlauchs (118) in den absorbierenden Abschnitt (134) integriert ist.

3. Flüssigkeitssammelpad nach einem der Ansprüche 1-2, wobei der absorbierende Abschnitt (134) Folgendes enthält:
mindestens eine Schicht, die hydrophobe Kügelchen (138) enthält;
mindestens eine Schicht, die ein absorbierendes Material (140) enthält; und
eine Netzschicht (142), die zwischen der mindestens einen Schicht, die die hydrophoben Kügelchen (138) enthält und der mindestens einen Schicht, die ein absorbierendes Material (140) enthält, angeordnet ist, wobei der zweite Abschnitt (118b) des Schlauchs (118) innerhalb der mindestens einen Schicht aus hydrophoben Kügelchen (138) angeordnet ist.

4. Flüssigkeitssammelpad nach einem der Ansprüche 1-3, wobei der Flüssigkeitssammelbehälter (120) aus einem auslaufsicheren Material gefertigt ist und von dem Kissen (112) entfernbar und austauschbar ist.

5. Flüssigkeitssammelpad nach Anspruch 1, ferner umfassend einen Geruchsneutralisator (144), der so konfiguriert ist, dass er einen Geruch der Flüssigkeit zumindest teilweise neutralisiert.

6. Tragbares Flüssigkeitssammelsystem, umfassend:
eine Flüssigkeitssammelvorrichtung (114), die dazu konfiguriert ist, mindestens in der Nähe einer Harnröhre eines Benutzers positioniert zu werden;
das Flüssigkeitssammelpad (110) nach einem der Ansprüche 1-5, wobei der Behälter innerhalb des Kissens (112) angeordnet ist; und
eine Pumpe (116), die in Fluidkommunikation mit dem Flüssigkeitssammelpad (110) und der Flüssigkeitssammelvorrichtung (114) steht, wobei die Pumpe (116) so konfiguriert ist, dass sie Flüssigkeit aus der Flüssigkeitssammelvorrichtung (114) durch den Schlauch (118) und in den Flüssigkeitssammelbehälter (120) zieht.

7. Flüssigkeitssammelsystem nach Anspruch 6, wobei der zweite Abschnitt (118b) des Schlauchs (118) in den absorbierenden Abschnitt (134) integriert ist.

8. Flüssigkeitssammelsystem nach Anspruch 6 oder 7, wobei der zweite Abschnitt (118b) des Schlauchs (118) Perforationen (136) beinhaltet, die verteilt sind, um die Flüssig gleichmäßig in dem Flüssigkeitssammelbehälter (120) zu verteilen, wenn die Flüssigkeit durch den zweiten Abschnitt (118b) strömt.

9. Tragbares Flüssigkeitssammelsystem nach einem der Ansprüche 6-8, das ferner einen Kissenbezug (124) umfasst, wobei das Flüssigkeitssammelpad (110) innerhalb des Kissenbezugs (124) angeordnet ist.

10. Tragbares Flüssigkeitssammelsystem nach einem der Ansprüche 6-9, wobei die Pumpe (116) mit dem Schlauch (118) zwischen der Flüssigkeitssammelvorrichtung (114) und dem Flüssigkeitssammelbehälter (120) gekoppelt ist.

11. Tragbares Flüssigkeitssammelsystem nach Anspruch 6, ferner umfassend einen Sensor (132), der mit dem Flüssigkeitssammelbehälter (120) gekoppelt ist und dazu konfiguriert ist, mindestens eine Eigenschaft anzugeben, die sich auf das Flüssigkeitssammelsystem (100) bezieht.

12. Tragbares Flüssigkeitssammelsystem nach Anspruch 11, wobei der Sensor (132) dazu konfiguriert ist, eine Eigenschaft zu erfassen, die ein Volumen der Flüssigkeit in dem Flüssigkeitssammelbehälter (120) anzeigt.

13. Tragbares Flüssigkeitssammelsystem nach Anspruch 6, ferner umfassend eine Anzeigetafel, wobei die Anzeigetafel mindestens entweder einen Alarm, eine Anzeige, einen Sättigungs- oder Volumenstatus eines Flüssigkeitssammelbehälters oder einen Pumpenbetriebsstatus beinhaltet.

14. Verfahren zum Zusammenbauen eines tragbaren Flüssigkeitssammelsystems, wobei das Verfahren Folgendes umfasst:
abnehmbares Befestigen eines Flüssigkeitssammelpads (110) an mindestens einem von entweder einem Stuhl, einem Rollstuhl (102) oder einem Bett;
Positionieren einer Flüssigkeitssammelvorrichtung (114) zumindest in der Nähe einer Harnröhre eines Benutzers;
fluidisches Koppeln der Flüssigkeitssammelvorrichtung (114) mit dem Flüssigkeitssammelpad (110), wobei das Flüssigkeitssammelpad (110) Folgendes beinhaltet:
ein Kissen (112);
einen Flüssigkeitssammelbehälter (120) mit einem darin angeordneten absorbierenden Abschnitt (134), wobei der Flüssigkeitssammelbehälter (120) so konfiguriert ist, dass er innerhalb des Kissens (112) angeordnet werden kann; und
einen Schlauch (118) mit einem ersten Abschnitt (118a), der so konfiguriert ist, dass er mit einer Flüssigkeitssammelvorrichtung (114) gekoppelt werden kann, und einem zweiten Abschnitt (118b), der innerhalb des Flüssigkeitssammelbehälters (120) angeordnet ist, wobei der zweite Abschnitt (118b) des Schlauchs (118) perforiert ist und in einer serpentinenartigen Anordnung durch den Flüssigkeitssammelbehälter (120) angeordnet ist; und
Koppeln einer Pumpe (116) mit dem ersten Abschnitt (118a) des Schlauchs (118), wobei die Pumpe (116) so konfiguriert ist, dass sie Flüssigkeit aus der Flüssigkeitssammelvorrichtung (114) durch den Schlauch (118) und in den Flüssigkeitssammelbehälter (120) zieht.

15. Verfahren nach Anspruch 14, wobei das abnehmbare Befestigen eines Flüssigkeitssammelsystems an mindestens einem von entweder einem Stuhl, einem Rollstuhl (102) oder einem Bett das Positionieren des Flüssigkeitssammelbehälters (120) innerhalb des Kissens (112) und das Platzieren des Kissens (112) innerhalb eines Kissenbezugs (124) umfasst, wobei der Kissenbezug (124) das Flüssigkeitssammelpad (110) innerhalb einer Tasche umschließt; und
Entfernen und Austauschen des Flüssigkeitssammelpads (110), wenn ein Volumen der Flüssigkeit in dem Flüssigkeitssammelbehälter (120) ein vorbestimmtes Volumen erreicht oder überschritten hat.

## Revendications

1. Tampon de collecte de fluide à utiliser avec un dispositif de collecte de fluide pour collecter le fluide d'un sujet, le tampon de collecte de fluide (110) comprenant :
un coussin (112) ;
un récipient de collecte de liquide (120) incluant une partie absorbante (134), dans lequel le récipient de collecte de liquide (120) est configuré pour être placé à l'intérieur du coussin (112) ; et
un tube (118) ayant une première partie (118a) configurée pour s'accoupler au dispositif de collecte de fluide (114) et une deuxième partie (118b) disposée à l'intérieur du récipient de collecte de fluide (120), dans lequel la deuxième partie (118b) du tube (118) est perforée ; **caractérisé en ce que** la deuxième partie (118b) du tube (118) est disposée en serpentin à travers le récipient de collecte de fluide (120).

2. Tampon de collecte de liquide de la revendication 1, dans lequel la deuxième partie (118b) du tube (118) est intégrée dans la partie absorbante (134).

3. Tampon de collecte des fluides de l'une des revendications 1-2, dans lequel la partie absorbante (134) inclut :
au moins une couche incluant des billes hydrophobes (138) ;
au moins une couche incluant un matériau absorbant (140) ; et
une couche de filet (142) disposée entre au moins une couche incluant les billes hydrophobes (138) et au moins une couche incluant un matériau absorbant (140), dans laquelle la deuxième partie (118b) du tube (118) est disposée à l'intérieur d'au moins une couche de billes hydrophobes (138).

4. Tampon de collecte des fluides de l'une des revendications 1 à 3, dans lequel le récipient de collecte des fluides (120) est fabriqué dans un matériau étanche et est amovible du coussin (112) et remplaçable.

5. Tampon de collecte de fluide de la revendication 1, comprenant en outre un neutralisateur d'odeur (144) configuré pour neutraliser au moins partiellement l'odeur du fluide.

6. Système portable de collecte des fluides, comprenant
un dispositif de collecte d'urine (114) configuré pour être positionné au moins à proximité de l'urètre d'un utilisateur ;
le coussin collecteur de fluide (110) selon l'une quelconque des revendications 1 à 5, dans lequel le récipient est disposé à l'intérieur du coussin (112) ; et
une pompe (116) en communication fluidique avec le tampon de collecte des fluides (110) et le dispositif de collecte des fluides (114), la pompe (116) étant configurée pour aspirer le fluide du dispositif de collecte des fluides (114) à travers le tube (118) et dans le récipient de collecte des fluides (120).

7. Système de collecte des fluides de la revendication 6, dans lequel la deuxième partie (118b) du tube (118) est intégrée dans la partie absorbante (134).

8. Système de collecte de fluide des revendications 6 ou 7, dans lequel la deuxième partie (118b) du tube (118) inclut des perforations (136) qui sont réparties pour disperser le fluide uniformément dans le récipient de collecte de fluide (120) lorsque le fluide s'écoule à travers la deuxième partie (118b).

9. Système portable de collecte des fluides de l'une des revendications 6 à 8 comprend en outre une housse de coussin (124), dans laquelle le tampon de collecte des fluides (110) est disposé dans la housse de coussin (124).

10. Système portable de collecte de fluides de l'une des revendications 6 à 9, dans lequel la pompe (116) est couplée au tube (118) entre le dispositif de collecte de fluides (114) et le récipient de collecte de fluides (120).

11. Système portable de collecte des fluides de la revendication 6, comprenant en outre un capteur (132) couplé au récipient de collecte des fluides (120) configuré pour indiquer au moins une propriété relative au système de collecte des fluides (100).

12. Système portable de collecte de fluide de la revendication 11, dans lequel le capteur (132) est configuré pour détecter une caractéristique indiquant un volume de fluide dans le récipient de collecte de fluide (120).

13. Système portable de collecte des fluides de la revendication 6 comprend en outre un panneau indicateur, dans lequel le panneau indicateur inclut au moins une alarme, un affichage, un état de saturation ou de volume du récipient de collecte des fluides, ou un état de fonctionnement de la pompe.

14. Méthode d'assemblage d'un système portable de collecte de fluides, consistant à
fixer de manière amovible un coussin collecteur de liquide (110) à au moins une chaise, un fauteuil roulant (102) ou un lit ;
positionner un dispositif de collecte de liquide (114) au moins à proximité de l'urètre d'un utilisateur ;
coupler fluidiquement le dispositif de collecte des fluides (114) au tampon de collecte des fluides (110), le tampon de collecte des fluides (110) incluant :
un coussin (112) ;
un récipient de collecte de liquide (120) incluant une partie absorbante (134), dans lequel le récipient de collecte de liquide (120) est configuré pour être placé à l'intérieur du coussin (112) ; et
un tube (118) ayant une première partie (118a) configurée pour s'accoupler à un dispositif de collecte de fluide (114) et une seconde partie (118b) disposée à l'intérieur du récipient de collecte de fluide (120), dans lequel la seconde partie (118b) du tube (118) est perforée et est disposée en serpentin à travers le récipient de collecte de fluide (120) ; et
coupler une pompe (116) à la première partie (118a) du tube (118), la pompe (116) étant configurée pour aspirer le fluide du dispositif de collecte de fluide (114) à travers le tube (118) et dans le récipient de collecte de fluide (120).

15. Méthode de la revendication 14, dans laquelle la fixation amovible d'un système de collecte de liquide à au moins une chaise, un fauteuil roulant (102) ou un lit inclut le positionnement du récipient de collecte de liquide (120) dans le coussin (112) et le placement du coussin (112) dans une housse de coussin (124), dans laquelle la housse de coussin (124) enferme le tampon de collecte de liquide (110) à l'intérieur d'une poche ; et
retirer et remplacer le tampon de collecte de fluide (110) lorsqu'un volume de fluide dans le récipient de collecte de fluide (120) a atteint ou dépassé un volume prédéterminé.
